# EUROPEAN PATENT APPLICATION

(11) **EP 2 333 050 A1**
(43) Date of publication of application: **15.06.2011**
(21) Application number: 09811587.6
(22) Date of filing: 04.09.2009
(51) Int. Cl.: C12N 5/10, A01K 67/027, C12N 15/09, C12P 21/08

(54) **B CELL-DERIVED IPS CELLS AND APPLICATION THEREOF**

(30) Priority: 04.09.2008 JP 2008227325
(71) Applicant: Riken, Wako-shi, Saitama 351-0198 (JP)
(72) Inventor: WATARAI, Hiroshi, Yokohama-shi Kanagawa 230-0045 (JP); IKAWA, Tomokatsu, Yokohama-shi Kanagawa 230-0045 (JP); ISHIKAWA, Fumihiko, Yokohama-shi Kanagawa 230-0045 (JP); KAWAMOTO, Hiroshi, Kanagawa 230-0045 (JP); KOSEKI, Haruhiko, Kanagawa 230-0045 (JP); TANIGUCHI, Masaru, Kanagawa 230-0045 (JP)
(74) Representative: Woods, Geoffrey Corlett
(86) International application number: PCT/JP2009/065534
(87) International publication number: WO 2010/027062

(57) **Abstract**

Provided are a B cell-derived iPS cell generated using a convenient technique, a technology for providing a human antibody at low cost using the iPS cell, an immunologically humanized mouse prepared using cells differentiated from the iPS cell, and the like. Also provided are a cloned cell obtained by contacting a B cell with nuclear reprogramming factors excluding C/EBPα and Pax5 expression inhibiting substances, particularly nucleic acids that encode Oct3/4, Sox2, Klf4 and c-Myc, wherein the cloned cell has an immunoglobulin gene rearranged therein and possesses pluripotency and replication competence (B-iPS cell). Still also provided are a method of producing a monoclonal antibody against a specified antigen, comprising recovering an antibody from a culture of B cells obtained by differentiating a B-iPS cell derived from a B cell immunized with the specified antigen, and a method of generating an immunologically humanized mouse, comprising transplanting to an immunodeficient mouse a human immunohematological system cell obtained by differentiating a B-iPS cell.

SEQUENCE LISTING

<110>  RIKEN

<120>  B Cell-derived iPS Cells and Use Thereof

<130>  091446

<150>  JP 2008-227325
<151>  2008-9-4

<160>  12

<170>  PatentIn version 3.5

<210>  1
<211>  27
<212>  DNA
<213>  Artificial Sequence

<220>
<223>  Primer

<400>  1
mtttttgtsa agggatctac tactgtg                                          27


<210>  2
<211>  25
<212>  DNA
<213>  Artificial Sequence

<220>
<223>  Primer

<400>  2
ctcacaagag tccgatagac cctgg                                            25


<210>  3
<211>  24
<212>  DNA
<213>  Artificial Sequence

<220>
<223>  Primer

<400>  3
tccaaggcgc ttctcacctc agtc                                             24


<210>  4
<211>  24
<212>  DNA
<213>  Artificial Sequence

<220>
<223>  Primer

<400>  4
cccagcagat tctcagtcca acag                                             24

<210>  5
<211>  24
<212>  DNA
<213>  Artificial Sequence

<220>
<223>  Primer

<400>  5
gcatgggctg aggctgatcc atta                                              24


<210>  6
<211>  25
<212>  DNA
<213>  Artificial Sequence

<220>
<223>  Primer

<400>  6
gaasamcctg twcctgcaaa tgasc                                             25


<210>  7
<211>  25
<212>  DNA
<213>  Artificial Sequence

<220>
<223>  Primer

<400>  7
carcacagcc twcatgcarc tcarc                                             25


<210>  8
<211>  25
<212>  DNA
<213>  Artificial Sequence

<220>
<223>  Primer

<400>  8
actgarcatc ascaaggaca aytcc                                             25


<210>  9
<211>  22
<212>  DNA
<213>  Artificial Sequence

<220>
<223>  Primer

<400>  9
ttatctggtg gtttcttcca gc                                                22

<210>  10
<211>  20
<212>  DNA
<213>  Artificial Sequence

<220>
<223>  Primer

<400>  10
gcacctgtgg ggaagaaact                                                    20


<210>  11
<211>  24
<212>  DNA
<213>  Artificial Sequence

<220>
<223>  Primer

<400>  11
cagagttcca tttcagaacc tagc                                               24


<210>  12
<211>  25
<212>  DNA
<213>  Artificial Sequence

<220>
<223>  Primer

<400>  12
tgagagctgt ctcctactat cgatt                                              25

## Description

### [Technical Field]

The present invention relates to a B cell-derived induced pluripotent stem (hereinafter referred to as iPS) cell, a method of producing the same, and use applications thereof.

### [Background Art]

iPS cells possess capabilities of differentiation and tissue formation equivalent to those of embryonic stem cells (ES cells). Because of inducibility from human primary culture cells, iPS cells are cells potentially possessing the capability of playing a central role in regenerative medicine. Yamanaka et al. established an iPS cell that possesses pluripotency as do ES cells by transferring four factors (Oct3/4, Sox2, Klf4, c-Myc) to mouse embryonic fibroblasts (MEF) (Non-patent Document 1). In addition to MEF, they succeeded in establishing mouse iPS cells from other various cells (Non-patent Documents 2 and 3), organs (Non-patent Document 4) and the like. Furthermore, in humans as well, establishment of iPS cells from human somatic cells using the same technique was reported (Non-patent Documents 5-8).

An aspect that can be a major barrier to ensuring the efficacy and safety of iPS cells in the context of their clinical application resides in the diversity thereof. It is speculated that the function of iPS cells differs among different lines depending on the individual's genetic background, the type of cells, the degree of reprogramming, the stage of ontogeny at which the cells are immortalized, and the like. In fact, even in mouse ES cells, the gene expression pattern differs widely depending on the genetic background, and the differentiation competence varies widely among different lines. It has already been found by Yamanaka et al. that in human iPS cells as well, the gene expression pattern differs widely among different lines (Non-patent Document 5). Therefore, it is anticipated that the differentiation competence and tumorigenesis tendency vary considerably among different iPS cells.
Also, there is a room for further improvement in the nuclear reprogramming protocol; various improved protocols have been reported (Non-patent Documents 9-14).

The immunohematological system has long been positioned as a subject of regenerative medicine or cytotherapy, from blood transfusion to bone marrow transplantation and cord blood transplantation, and these therapies have been shown to be substantially effective. It has also been shown that in mice and humans, differentiation of a variety of immunohematological system cells can be induced from ES cells. This shows that induction of immunohematological system cells from iPS cells would be potentially effective as a therapy within the conventional framework.

B cells, which are in the series of lymphocytes constituting the immune system, are antibody-producing cells, originating from bone marrow-derived hematopoietic stem cells and differentiating and maturing in the spleen. In this process, H-chain / L-chain gene recombination is induced, and each clone exhibits its characteristic antigen specificity. In the prior art, myeloma and B cells have been cell-fused to yield hybridomas, which are screened to establish antigen-specific monoclonal antibodies. In the medical field, in particular, a large number of antibodies that target antigens such as cell surface antigens, cancer antigens, cytokines, and growth factors, mainly mouse antibodies, chimeric antibodies, humanized antibodies, and human antibodies, have already been launched as antibody pharmaceuticals in the market, or are under clinical studies. For example, Herceptin (a cancer antigen Her2-targeting therapeutic drug for metastatic breast cancer, Roche/Genentech), rituximab (a CD20 antigen-targeting therapeutic drug for non-Hodgkin lymphoma, Genentech) and the like have already been shown to be actually appreciable in terms of efficacy, safety, mitigation of adverse reactions and the like.
However, all the antibody pharmaceuticals being currently distributed in the market are created using gene engineering techniques, posing a major problem of forcing up medical expenses due to their extremely high prices. Although an attempt has been made to prepare a human antibody from human B cells using the in vitro immunization method and a virus-based technology for cell immortalization or human-human (or mouse) hybridoma generation, this has not yet been in practical use.

It is expected that by reprogramming human B cells that produce an antibody specific for a certain antigen, and then allowing them to differentiate and mature again, an antibody-producing cell clone can be expanded in large amounts without using gene engineering techniques. However, finally differentiated cells are less easy to reprogram, compared with undifferentiated cells; iPS cells cannot be induced with what are called the four factors or three factors (Oct3/4, Sox2, Klf4) only, their induction often requiring the use of another gene as a nuclear reprogramming factor (Non-patent Document 3). However, increasing the number of transgenes is feared to intensity safety concerns, including the potential tumorigenesis of the cells differentiated from iPS cells.

### [prior art references]

### [non-patent documents]

non-patent document 1: Cell. 2006 Aug 25; 126(4): 663-676.
non-patent document 2: Nature. 2007 Jul 19; 448(7151): 313-317. Epub2007 Jun 6.
non-patent document 3: Cell. 2008 Apr 18; 133(2): 250-264. Erratum in: Cell. 2008 Jul 25; 134(2): 365.
non-patent document 4: Science. 2008 Aug 1; 321(5889): 699-702. Epub2008 Feb 14.
non-patent document 5: Cell. 2007 Nov 30; 131(5): 861-872. non-patent document 6: Science. 2007 Dec 21; 318(5858): 1917-1920. Epub 2007 Nov 20.
non-patent document 7: Nature. 2008 Jan 10; 451(7175): 141-146. Epub 2007 Dec 23.
non-patent document 8: Proc Natl Acad Sci USA. 2008 Feb 26; 105(8): 2883-2888. Epub 2008 Feb 15.
non-patent document 9: Nat Biotechnol. 2008 Jan; 26(1): 101-106. Epub 2007 Nov 30.
non-patent document 10: Nat Biotechnol. 2008 Aug; 26(8): 916-24. Epub 2008 Jul 1.
non-patent document 11: Utikal JS, Arnold K, Jaenisch R, Hochedlinger K. Reprogramming of neural progenitor cells into iPS cells in the absence of exogenous Sox2 expression. Stem Cells. Epub 2008 Jul 17.
non-patent document 12: Nature. 2008 Jul 31; 454(7204): 646-650. Epub2008 Jun 29.
non-patent document 13: Cell Stem Cell. 2008 Jun 5; 2(6): 525-528.
non-patent document 14: Curr Biol. 2008 Jun 24; 18(12): 890-894. Epub 2008 May 22.

### [Summary of the Invention]

### [Problems to Be Solved by the Invention]

It is an object of the present invention to provide an iPS cell derived from a B cell using a convenient technique, and to provide a human antibody at low cost using the iPS cell. It is another object of the present invention to provide an immunocyte therapeutic agent, an immunologically humanized mouse and the like prepared using cells differentiated from the iPS cell.

### [Means of Solving the Problems]

The present inventors conducted extensive investigations to solve the above-described problems, and unexpectedly succeeded in establishing a cell having immunoglobulin genes rearranged therein, and possessing proliferation competence and pluripotency by introducing only the four factors Oct3/4, Sox2, Klf4, and c-Myc into a mouse spleen-derived B cell using a retrovirus. The present inventors conducted further investigations based on these findings, and have developed the present invention.

Accordingly, the present invention relates to the following:
[1] A cloned cell obtained by contacting a B cell with nuclear reprogramming factors that do not include C/EBPα and Pax5 expression inhibiting substances, wherein the cloned cell has immunoglobulin genes rearranged therein and possesses pluripotency and replication competence.
[2] The cell described in [1] above, wherein the nuclear reprogramming factors are Oct3/4, Sox2, Klf4 and c-Myc or nucleic acids that encode the same, or Oct3/4, Sox2 and Klf4 or nucleic acids that encode the same.
[3] The cell described in [1] or [2] above, wherein the B cell is of human derivation.
[4] The cell described in any one of [1] to [3] above, wherein the B cell is immunized with a specified antigen.
[5] A method of producing a monoclonal antibody against a specified antigen, comprising recovering an antibody from a culture of B cells obtained by differentiating the cell described in [4] above.
[6] A method of generating an immunologically humanized mouse, comprising transplanting human immunohematological system cells obtained by differentiating the cell described in [3] above to an immunodeficient mouse.
[7] An immunologically humanized mouse obtained by the method described in [6] above.

### Effect of the Invention

Monoclonal antibodies prepared via the B cell-derived iPS cell of the present invention can be produced at extremely low cost because no gene engineering technology is used. Also, by using a humanized mouse generated by utilizing the B cell-derived iPS cell of the present invention, it is possible to evaluate the direct effect and adverse reactions to the human immune system of a novel drug that acts directly on the immune system in the preclinical phase.

### Brief Description of the Drawings

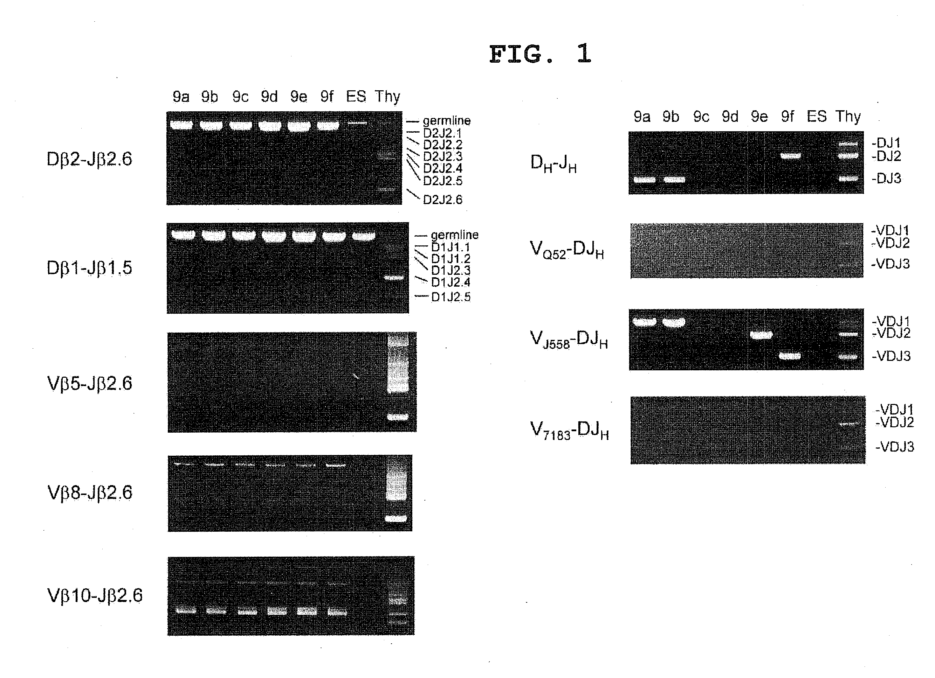

Fig. 1 is a drawing showing the presence or absence of the rearrangement of the BCR gene in six established B-iPS cell clones (9a, 9b, 9c, 9d, 9e, 9f).

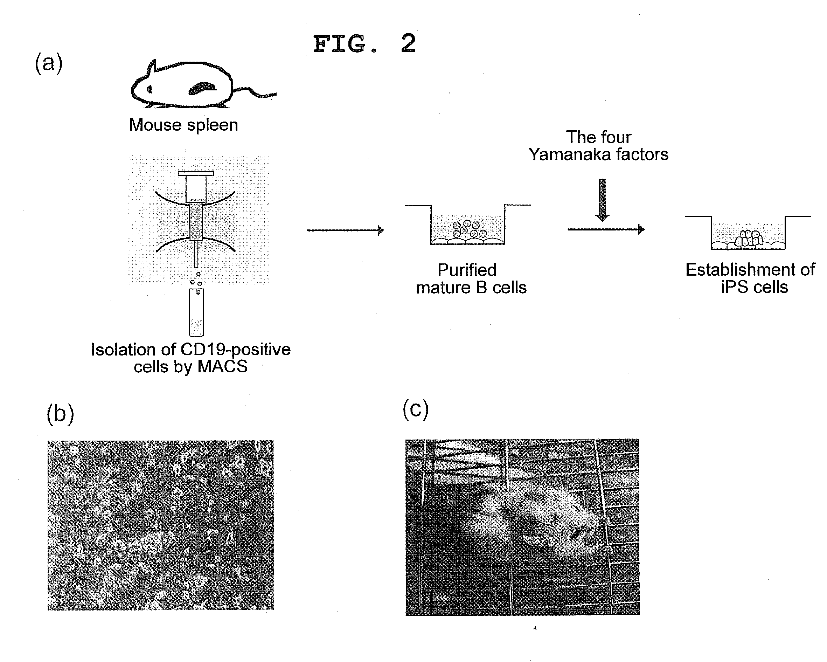

Fig. 2 is a drawing showing the generation of iPS cells from purified B cells. (a) shows an outline of the procedure; (b) shows an example of the mature B cell-derived iPS cells obtained; (c) shows a chimeric mouse wherein tissues derived from the iPS cells are co-present, obtained by injecting the iPS cells obtained into an embryo of a white mouse.

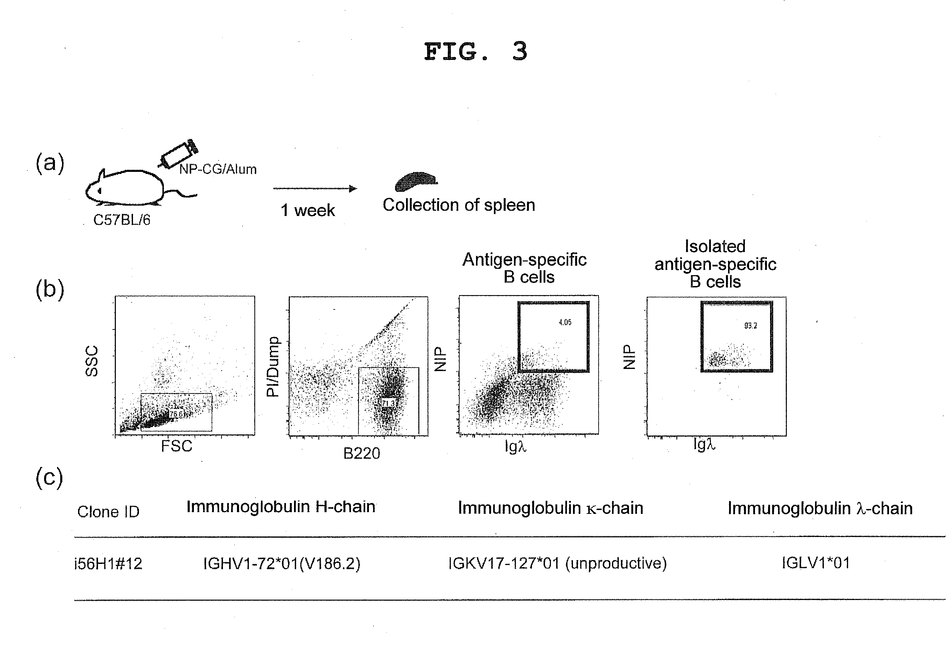

Fig. 3 is a drawing showing the establishment of iPS cells from antigen-specific B cells. (a) shows the procedure of mouse immunization; (b) shows the FACS profiles utilized to isolate the antigen-specific B cells used to establish the iPS cells; (c) shows the results of DNA sequencing of the antigen-specific B cell-derived iPS cell clone i56H1#12.

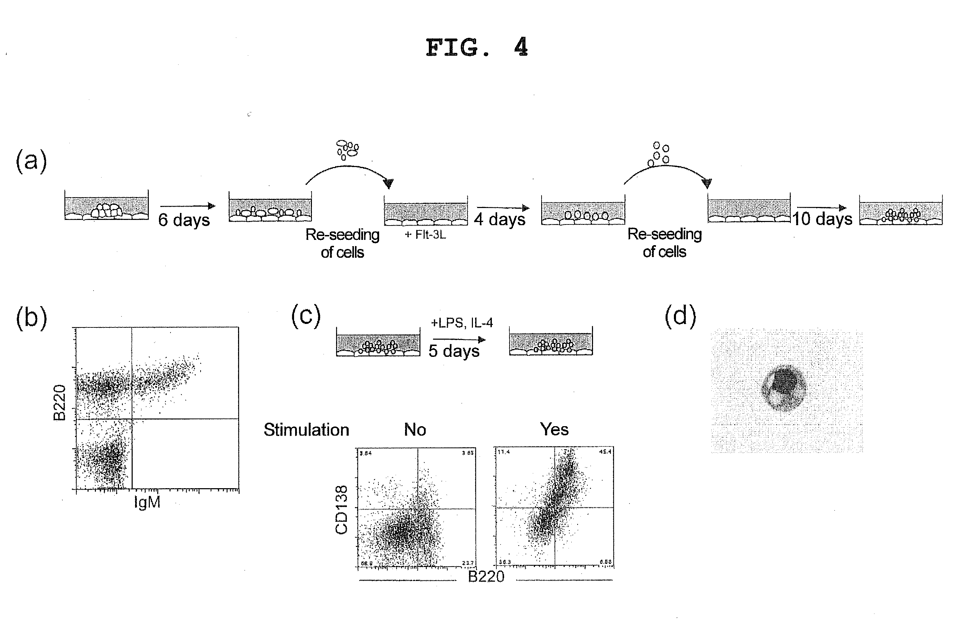

Fig. 4 is a drawing showing the generation of antibody-producing cells from iPS cells. (a) shows the culturing procedure for generating B cells from mouse mesenchymal cell-derived iPS cells; (b) shows the FACS profile of B cells induced from iPS cells; (c) shows the induction of antibody-producing cells from B cells, the upper panel showing the culturing procedure therefor, the lower panel showing FACS profiles obtained from B cells without (left in the lower panel) or with (right in the lower panel) stimulation with LPS (25 mg/ml) and IL-4 (10 ng/ml); (d) shows an example of antibody-producing cells obtained from iPS cells (plasma cells).

### [Modes for Embodying the Invention]

The present invention provides a B cell-derived cloned iPS cell having immunoglobulin genes rearranged therein, and possessing pluripotency and replication competence (hereinafter referred to as "B-iPS cell"). As used herein, "an iPS cell" refers to a cell that has acquired pluripotency and replication competence conferred artificially by contacting a somatic cell with a nuclear reprogramming factor. Here, "pluripotency" means the ability to differentiate into a plurality of series of immunohematological system cells such as B cells, T cells, erythrocytes, macrophages and progenitor cells thereof, as well as into one or more cell series other than the immunohematological system, and is distinguished from multipotency in hematopoietic stem cells and multipotent progenitor cells. "Replication competence" means the ability for a cell to continue to expand in a particular environment (for example, conditions suitable for culturing ES cells) while retaining the above-described "pluripotency".

The B-iPS cell of the present invention can be established by contacting a B cell with nuclear reprogramming factors not including C/EBPα and Pax5 expression inhibiting substances. As used herein, the term "B cell" is used with a meaning encompassing not only mature B cells, but also finally differentiated plasma cells and optionally chosen B progenitor cells excluding pre-B cells and progenitor cells thereof. In terms of the expression of cell surface markers, the B-iPS cell of the present invention is characterized by IgM⁺, IgD⁺, IgG⁺, CD19⁺, B220⁺, CD24⁺, CD43⁻, CD25⁻, c-kit⁻, IL-7R⁻ and the like. B cells can be isolated from the spleen, lymph node, peripheral blood, cord blood and the like by a method known per se, for example, flow cytometry using an antibody against each of the above-described various cell surface markers and a cell sorter. In the case of mice, it is preferable to collect B cells from the spleen or lymph node, wherein the abundance ratio of B cells is high; however, in the case of humans, it is desirable, from the viewpoint of low invasiveness and the ease of preparation, that the B cells be prepared from peripheral blood, cord blood and the like.

The B cell used in the present invention may be derived from any animal species that permits the establishment of B-iPS cells by contacting the B cell with nuclear reprogramming factors; specifically, those of human or mouse derivation can be mentioned, and human-derived B cells are preferred. The human or mouse that serves as the source of B cells collected is not particularly limited; however, when the B-iPS cells obtained are to be used for regenerative medicine in humans,
it is particularly preferable, from the viewpoint of prevention of graft rejection, that the B cells be collected from the patient or from another person having the same HLA type as that of the patient. When the B-iPS cells obtained are not to be administered (transplanted) to a human, but used as, for example, a source of cells for screening for evaluating a patient's drug susceptibility or the presence or absence of adverse reactions, it is necessary to collect the B cells from the patient or from another person with the same genetic polymorphism correlating with the drug susceptibility or adverse reactions.

The B cells prepared from the spleen, lymph node, peripheral blood, cord blood and the like by the above-described method may be immediately contacted with nuclear reprogramming factors to induce B-iPS cells, or may also be preserved under freezing by a conventional method, thawed just before use, and cultured, and then contacted with nuclear reprogramming factors to induce B-iPS cells. Therefore, it is possible, for example, to preserve B cells prepared from the recipient's own spleen, lymph node, peripheral blood, cord blood and the like under freezing for a long time while he or she is healthy, to induce B-iPS cells from the B cells and auto-transplant cells, tissues and the like obtained by differentiation induction therefrom when cell/organ transplantation becomes necessary in a later year.

Preserved in the B-iPS cell of the present invention are immunoglobulin genes rearranged in the B cell from which the cell clone is derived. For this reason, in the mature B cells and plasma cells obtained by differentiating B-iPS cells induced from a B cell immunized with a certain antigen, a monoclonal antibody against the antigen is produced. Therefore, in a preferred embodiment of the present invention, the B cell used to prepare B-iPS cells has been immunized with a specified antigen in advance. Although the antigen used for immunization is not particularly limited, from the viewpoint of utilizing B-iPS cells as a source of antibody-producing cells for antibody pharmaceuticals, examples of the antigen include antigens that can be target molecules for antibody pharmaceuticals, for example, proteins such as cell surface antigens (e.g., CD20 and the like), cancer antigens (e.g., Her2, EGFR and the like), cytokines (e.g., IL-1-20, IFNα-γ, TNF and the like), and growth factors (e.g., EGF, TGF-α, PDGF, VEGF and the like), fragments thereof, sugars, nucleic acids, lipids and the like.

Regarding the method of immunizing B cells with an antigen, in the case of mice, a method may be used wherein the antigen is administered as it is alone, or along with a carrier or a diluent, by a method of administration such as intraperitoneal injection, intravenous injection, subcutaneous injection, or intradermal injection, at a site enabling antibody production, as in preparing an ordinary mouse monoclonal antibody. In order to increase antibody productivity upon the administration, Freund's complete adjuvant or Freund's incomplete adjuvant may be administered. Administration is normally performed about 1 to 10 times in total every 1 to 6 weeks. An individual exhibiting an elevated antibody titer is selected, the spleen or lymph node is collected 2 to 7 days after the final immunization, and B cells are recovered.

Since artificial immunization to humans is ethically difficult, it is preferable to use the in vitro immunization method. The in vitro immunization method can also be used preferably as a method for obtaining an antibody against an antigen that is unstable and difficult to prepare in large amounts, for the purpose of preparing a non-human animal-derived antibody, because there is the possibility of obtaining an antibody against an antigen for which antibody production is suppressed by ordinary immunization, as well as because it is possible to obtain an antibody with an amount of antigen on the nanogram to microgram order, and also because immunization completes in several days, and for other reasons.
The B cells used in the in vitro immunization method can be isolated from peripheral blood, cord blood, spleen, lymph node and the like of a human, mouse or the like, as described above. For example, in the case of mouse B cells, the spleen is extirpated from an about 4- to 12-week-old animal, and splenocytes are separated and washed with an appropriate medium (e.g., Dulbecco's modified Eagle medium (DMEM), RPMI1640 medium, Ham's F12 medium and the like), after which the splenocytes are suspended in an antigen-containing medium supplemented with fetal calf serum (FCS; about 5 to 20%) and cultured using a CO₂ incubator and the like for about 4 to 10 days. Examples of the antigen concentration include, but are not limited to, 0.05 to 5 µg. It is preferable to prepare a culture supernatant of thymocytes of an animal of the same strain (preferably at about 1 to 2 weeks of age) according to a conventional method, and to add the supernatant to the medium.
Since it is difficult to obtain a thymocyte culture supernatant in in vitro immunization of human cells, it is preferable to perform immunization by adding, to the medium, several kinds of cytokines such as IL-2, IL-4, IL-5, and IL-6, and if necessary, an adjuvant substance (e.g., muramyldipeptide and the like), along with the antigen.
A cell population exhibiting an elevated antibody titer is selected and immunized in vitro, after which the cells are cultured for 4 to 10 days and then recovered, and antibody-producing B cells are isolated.

In another preferred embodiment, B cells can be immunized by utilizing an immunodeficient mouse having human hematopoietic stem cells allowed to take to reproduce human hematopoiesis therein, that is, an immunologically humanized mouse. Human hematopoietic stem cells used for the transplantation include CD34⁺ cells and the like collected from cord blood, peripheral blood, bone marrow and the like. Examples of preferred recipient immunodeficient mice include severe combined immunodeficiency mice (SCID mice) that lack the potential for producing T cells and B cells, particularly NOD/SCID/β2 microglobulin knock-out mice (NOD/SCID/B2M), NOD/SCID/common γ-chain knock-out mice and the like, which do not have NK cell activity, and the like. Furthermore, it is desirable to use mice prepared by transfecting these mice with human HLA (histocompatibility antigen). For further details on preparing an immunologically humanized mouse using hematopoietic stem cells, Japanese Domestic Re-publication of PCT International Publication for Patent Application 2004-110139, for example, can be referenced to. Immunization with an antigen may be performed according to the method described above. For the purpose of promoting the generation of, and facilitating the recovery of, antigen-specific B cells in a humanized mouse, it is also possible to use a humanized mouse allowed to form an artificial lymph node. An artificial lymph node is a lymph tissue induced and formed by transplanting a piece of support with a three-dimensional structure under the renal coat of a mouse or elsewhere. For details, WO2007/069755 and Suematsu S et al., Nature Biotechnol 22: 1539-45 (2004) can be referenced to.

In the present invention, "a nuclear reprogramming factor" may be composed of any substance such as a proteinous factor(s) or a nucleic acid that encodes the same (including forms incorporated in a vector) or a low molecular compound, as far as it is a substance (a group of substances) capable of inducing cells possessing pluripotency and replication competence from a B cell. When the nuclear reprogramming factor is a proteinous factor or a nucleic acid that encodes the same, the following combinations, for example, are preferable (hereinafter, only the names for proteinous factors are shown) .
(1) Oct3/4, Klf4, Sox2, c-Myc (Sox2 is replaceable with Sox1, Sox3, Sox15, Sox17 or Sox18; Klf4 is replaceable with Klf1, Klf2 or Klf5; c-Myc is replaceable with T58A (active mutant), N-Myc, or L-Myc)
(2) Oct3/4, Klf4, Sox2
(3) Oct3/4, Klf4, c-Myc
(4) Oct3/4, Sox2, Nanog, Lin28
(5) Oct3/4, Klf4, c-Myc, Sox2, Nanog, Lin28
(6) Oct3/4, Klf4, Sox2, bFGF
(7) Oct3/4, Klf4, Sox2, SCF
(8) Oct3/4, Klf4, c-Myc, Sox2, bFGF
(9) Oct3/4, Klf4, c-Myc, Sox2, SCF
When bearing in mind the use of the B-iPS cells obtained for therapeutic purposes, the combination of the three factors Oct3/4, Sox2 and Klf4 is preferable out of these combinations. Meanwhile, when not bearing in mind the use of the B-iPS cells for therapeutic purposes (for example, use as a source of antibody-producing cells to be prepared, use as a research tool for drug discovery screening and the like, and the like), the four factors Oct3/4, Klf4, Sox2 and c-Myc or the five factors consisting of the same four factors and Lin28 or Nanog are preferred. Particularly preferably, the nuclear reprogramming factors in the present invention are the four factors Oct3/4, Klf4, Sox2 and c-Myc.

In the present invention, C/EBPα and Pax5 expression inhibiting substances are not included in the nuclear reprogramming factors. Here, "C/EBPα" includes not only proteinous factors, but also nucleic acids that encode the same. "Pax5 expression inhibiting substances" include antisense nucleic acids, siRNAs, shRNAs, and ribozymes against Pax5 and expression vectors that encode the same and the like. Because of the obviation of these factors in the nuclear reprogramming step, the present invention makes it possible to acquire B-iPS cells more conveniently, and to reduce the potential tumorigenesis in the cells and tissues differentiation-induced from the B-iPS cells.

Information on the mouse and human cDNA sequences of the aforementioned proteinous factors is available with reference to the NCBI accession numbers mentioned in WO 2007/069666 (in the publication, Nanog is described as ECAT4; mouse and human cDNA sequence information on Lin28 can be acquired by referring to the following NCBI accession numbers NM_145833 and NM_024674, respectively). Those skilled in the art are easily able to isolate these cDNAs. A proteinous factor for use as a nuclear reprogramming factor can be prepared by inserting the cDNA obtained into an appropriate expression vector, transferring the vector into a host cell, culturing the cell, and recovering the recombinant proteinous factor from the culture obtained. Meanwhile, when the nuclear reprogramming factor used is a nucleic acid that encodes a proteinous factor, the cDNA obtained is inserted into a viral or plasmid vector to construct an expression vector, and the vector is subjected to the step of nuclear reprogramming.

Contact of a nuclear reprogramming factor with B cell can be achieved using a method known per se for protein transfer into cells when the substance is a proteinous factor. Such methods include, for example, the method using a protein transfer reagent, the method using a protein transfer domain (PTD) fusion protein, the microinjection method and the like. Protein transfer reagents are commercially available, including those based on a cationic lipid, such as BioPOTER Protein Delivery Reagent (Gene Therapy Systems), Pro-Ject^{™} Protein Transfection Reagent (PIERCE) and ProVectin (IMGENEX); those based on a lipid, such as Profect-1 (Targeting Systems); those based on a membrane-permeable peptide, such as Penetrain Peptide (Q biogene) and Chariot Kit (Active Motif), and the like. The transfer can be achieved per the protocols attached to these reagents, a common procedure being as described below. A nuclear reprogramming factor is diluted in an appropriate solvent (e.g., a buffer solution such as PBS or HEPES), a transfer reagent is added, the mixture is incubated at room temperature for about 5 to 15 minutes to form a complex, this complex is added to cells after exchanging the medium with a serum-free medium, and the cells are incubated at 37°C for one to several hours. Thereafter, the medium is removed and replaced with a serum-containing medium.
Developed PTDs include those using transcellular domains of proteins such as drosophila-derived AntP, HIV-derived TAT, and HSV-derived VP22. A fusion protein expression vector incorporating a cDNA of a nuclear reprogramming factor and a PTD sequence is prepared to allow the recombinant expression
of the fusion protein, and the fusion protein is recovered for use in for transfer. This transfer can be achieved as described above, except that no protein transfer reagent is added.
Microinjection, a method of placing a protein solution in a glass needle having a tip diameter of about 1 µm, and injecting the solution into a cell, ensures the transfer of the protein into the cell.

However, taking into account the ease of transfer into B cell, a nuclear reprogramming factor is used preferably in the form of a nucleic acid that encodes a proteinous factor, rather than the factor as it is. The nucleic acid may be a DNA or an RNA, or a DNA/RNA chimera, and may be double-stranded or single-stranded. Preferably, the nucleic acid is a double-stranded DNA, particularly a cDNA.
A cDNA of a nuclear reprogramming factor is inserted into an appropriate expression vector comprising a promoter capable of functioning in a host B cell. Useful expression vectors include, for example, viral vectors such as retrovirus, lentivirus, adenovirus, adeno-associated virus and herpesvirus, plasmids for the expression in animal cells (e.g., pA1-11, pXT1, pRc/CMV, pRc/RSV, pcDNAI/Neo) and the like.
A kind of vector used can be chosen as appropriate according to the intended use of the iPS cells obtained. For example, adenovirus vector, plasmid vector, adeno-associated virus vector, retrovirus vector, lentivirus vector and the like can be used.

Examples of promoters used in expression vectors include the SRα promoter, the SV40 promoter, the LTR promoter, the CMV (cytomegalovirus) promoter, the RSV (Rous sarcoma virus) promoter, the MoMuLV (Moloney mouse leukemia virus) LTR, the HSV-TK (herpes simplex virus thymidine kinase) promoter and the like, with preference given to the MoMuLV LTR, the CMV promoter, the SRα promoter and the like.

The expression vector may contain as desired, in addition to a promoter, an enhancer, a polyA addition signal, a selection marker gene, a SV40 replication origin and the like. Examples of useful selection marker genes include the dihydrofolate reductase gene and the neomycin resistance gene.
An expression vector harboring a nucleic acid as a nuclear reprogramming factor can be transferred into a cell by a technique known per se according to the choice of the vector. In the case of a viral vector, for example, a plasmid containing the nucleic acid is introduced into an appropriate packaging cell (e.g., Plat-E cells) or a complementary cell line (e.g., 293-cells), the viral vector produced in the culture supernatant is recovered, and the vector is infected to the cell by a method suitable for the viral vector. Meanwhile, a plasmid vector can be transferred into a cell using the lipofection method, liposome method, electroporation method, calcium phosphate co-precipitation method, DEAE dextran method, microinjection method, gene gun method and the like.

When the nuclear reprogramming factor is a low-molecular compound, contact of the substance with B cells can be achieved by dissolving the substance at an appropriate concentration in an aqueous or non-aqueous solvent, adding the substance solution to a medium suitable for cultivation of B cells isolated from a human or mouse (for example, a minimal essential medium (MEM), Dulbecco's modified Eagle medium (DMEM), RPMI1640 medium, 199 medium, and F12 medium containing cytokines such as IL-2, IL-7, SCF, and Flt3 ligands, LPS, and about 5 to 20% fetal bovine serum, and the like) so that the nuclear reprogramming factor concentration will fall in a range that is sufficient to cause nuclear reprogramming in B cells and does not cause cytotoxicity, and culturing the cells for a given period. The nuclear reprogramming factor concentration varies depending on the kind of nuclear reprogramming factor used, and is chosen as appropriate over the range of about 0.1 nM to about 100 nM. Duration of contact is not particularly limited, as far as it is sufficient to achieve nuclear reprogramming of the cells; usually, the nuclear reprogramming factor may be allowed to be co-present in the medium until a positive colony emerges.

In recent years, various substances that improve the efficiency of establishment of iPS cells, which has traditionally been low, have been proposed one after another. When brought into contact with B cell together with the aforementioned nuclear reprogramming factors, these establishment efficiency improvers are expected to further raise the efficiency of establishment of B-iPS cells.
Examples of iPS cell establishment efficiency improvers include, but are not limited to, histone deacetylase (HDAC) inhibitors [for example, low-molecular inhibitors such as valproic acid (VPA) (Nat. Biotechnol., 26(7): 795-797 (2008)), trichostatin A, sodium butyrate, MC 1293, and M344; nucleic acid-based expression inhibiting agents such as siRNAs and shRNAs against HDAC (e.g., HDAC1 siRNA Smartpool (registered trademark) (Millipore), HuSH 29mer shRNA Constructs against HDAC1 (OriGene) and the like); and the like], G9a histone methyltransferase inhibitors [e.g., low-molecular inhibitors such as BIX-01294 (Cell Stem Cell, 2: 525-528 (2008)); nucleic acid-based expression inhibitors such as siRNAs and shRNAs against G9a (for example, G9a siRNA (human) (Santa Cruz Biotechnology) and the like; and the like], and the like. The nucleic acid-based expression inhibitors may be in the form of expression vectors harboring a DNA that encodes an siRNA or shRNA.

Contact of an iPS cell establishment efficiency improver with B cell can be achieved as described above for each of three cases: (a) the improver is a proteinous factor, (b) the improver is a nucleic acid that encodes the proteinous factor, and (c) the improver is a low-molecular compound.
An iPS cell establishment efficiency improver may be brought into contact with B cell simultaneously with a nuclear reprogramming factor, or either one may be contacted in advance, as far as the efficiency of establishment of B-iPS cells from B cell is significantly improved, compared with the absence of the improver. In an embodiment, for example, when the nuclear reprogramming substance is a nucleic acid that encodes a proteinous factor and the iPS cell establishment efficiency improver is a chemical inhibitor, the iPS cell establishment efficiency improver can be added to the medium after the cell is cultured for a given length of time after the gene transfer treatment, because the nuclear reprogramming substance involves a given length of time lag from the gene transfer treatment to the mass-expression of the proteinous factor, whereas the iPS cell establishment efficiency improver is capable of rapidly acting on the cell. In another embodiment, when a nuclear reprogramming factor and an iPS cell establishment efficiency improver are both used in the form of a viral vector or plasmid vector, for example, both may be simultaneously transferred into the cell.

The B cells separated from a human or mouse can also be pre-cultured using a medium known per se that is suitable for their cultivation (for example, a minimal essential medium (MEM), Dulbecco's modified Eagle medium (DMEM), RPMI1640 medium, 199 medium, and F12 medium containing cytokines such as IL-2, IL-7, SCF, and Flt3 ligands, and about 5 to 20% fetal bovine serum, and the like).
When a transfection reagent such as a cationic liposome, for example, is used in contacting with nuclear reprogramming factors (and an iPS cell establishment efficiency improver), it is sometimes preferable that the medium be previously replaced with a serum-free medium to prevent a reduction in the transfer efficiency. After the nuclear reprogramming factors (and iPS cell establishment efficiency improver) are contacted, the cells can be cultured under conditions suitable for the cultivation of, for example, ES cells. In the case of human cells, it is preferable that the cultivation be carried out with the addition of basic fibroblast growth factor (bFGF) as a differentiation suppressor to an ordinary medium. Meanwhile, in the case of mouse cells, it is desirable that Leukemia Inhibitory Factor (LIF) be added in place of bFGF. Usually, the cells are cultured in the co-presence of fetal-mouse-derived fibroblasts (MEFs) treated with radiation or an antibiotic to terminate the cell division thereof, as feeder cells. Usually, STO cells and the like are commonly used as MEFs, but for inducing iPS cells, SNL cells [McMahon, A.P. & Bradley, A. Cell 62, 1073-1085 (1990)] and the like are commonly used.

A candidate colony of B-iPS cells can be selected by a method with drug resistance and reporter activity as indicators, and also by a method based on visual examination of morphology. As an example of the former, a colony positive for drug resistance and/or reporter activity is selected using a recombinant B cell wherein a drug resistance gene and/or a reporter gene is targeted to the locus of a gene highly expressed specifically in pluripotent cells (e.g., Fbx15, Nanog, Oct3/4 and the like, preferably Nanog or Oct3/4). Meanwhile, examples of the latter method based on visual examination of morphology include the method described by Takahashi et al. in Cell, 131, 861-872 (2007). Although the method using reporter cells is convenient and efficient, it is desirable, from the viewpoint of safety, that colonies be selected by visual examination when the B-iPS cells are prepared for the purpose of applying to human treatment; even by visual morphological examination, a candidate colony of B-iPS cells can be selected well efficiently.

The identity of the cells of the selected colony as B-iPS cells can be confirmed by various testing methods known per se, for example, expressional analysis of ES cell-specific genes (for example, Oct3/4, Sox2, Nanog, Cripto, Dax1, ERas, Fgf4, Esg1, Rex1, Zfp296 and the like) and the like. To ensure higher accuracy, it is possible to transplant the selected cells to a mouse and confirm the formation of teratomas.

Confirmation of the derivation of the B-iPS cells from a B cell can be achieved by examining for the presence or absence of B cell receptor (BCR) gene rearrangement by genomic PCR as described in Example 2 below.

The B-iPS cells thus established can be used for various purposes. For example, by utilizing a method of differentiation induction reported to have been applied to ES cells, hematopoietic stem cells and the like, differentiation into various cells (e.g., immunohematological system cells such as B cells, plasma cells, T cells, NK cells, NKT cells, neutrophils, eosinophils, basophils, mast cells, and macrophages, cardiac muscle cells, retinal cells, nerve cells, vascular endothelial cells, insulin-secreting cells and the like), tissues, and organs from B-iPS cells can be induced.
For example, according to a method described in JP-A-2006-141356, B-iPS cells can be differentiated into B cells via hematopoietic stem cells.

In a preferred embodiment, the B-iPS cells are differentiated into mature B cells or plasma cells for utilization as antibody-producing cells. Accordingly, the present invention also provides a method of producing a monoclonal antibody against a specified antigen, comprising recovering an antibody from a culture of the B cells obtained by differentiating B-iPS cells prepared from B cells immunized with the specified antigen, by the above-described method.
When it is intended to mass-produce an antibody, it is preferable that the B-iPS cells have been expanded in sufficient amounts before inducing differentiation into B cells. As a medium for B-iPS cell proliferation, a medium in use for ES cell culture is generally usable.

Example methods of inducing differentiation from B-iPS cells to B cells include, but are not limited to, a method comprising co-cultivation with stromal cells (e.g., OP9 cells, S17 cells and the like) in a medium such as a minimal
essential medium (MEM), Dulbecco's modified Eagle medium (DMEM), RPMI1640 medium, 199 medium, or F12 medium containing cytokines such as IL-2, IL-4, IL-7, SCF, and Flt3 ligands, LPS, and about 5 to 20% fetal bovine serum for several weeks.

The mature B cells and plasma cells obtained are cultured according to a conventional method, and the desired monoclonal antibody is recovered from the culture supernatant. For separating and purifying the monoclonal antibody, ordinary protein separation and purification techniques can be used in combination; affinity column chromatography using an antigen-immobilized column and the like are particularly preferable.

When the monoclonal antibody obtained as described above is a therapeutic antibody, the antibody can be administered as a liquid as it is, or as an appropriate dosage form of pharmaceutical composition, to humans or other mammals (e.g., mice and the like) orally or parenterally (e.g., intravascular administration, subcutaneous administration and the like).
The pharmaceutical composition used for administration may contain both the above-described antibody and a pharmacologically acceptable carrier, diluent or excipient. Such a pharmaceutical composition is supplied in the form of a dosage form suitable for oral or parenteral administration.

As examples of the composition for parenteral administration, injections, suppositories and the like are used; the injections may include dosage forms such as intravenous injections, subcutaneous injections, intradermal injections, intramuscular injections and drip infusion injections. Such an injection can be prepared according to a publicly known method. An injection can be prepared by, for example, dissolving, suspending or emulsifying the above-described antibody in a sterile aqueous or oily solution in common use for injections. As examples of aqueous solutions for injection, physiological saline, an isotonic solution containing glucose or another auxiliary drug, and the like can be used, which may be used in combination with an appropriate solubilizer, for example, alcohol (e.g., ethanol), polyalcohol (e.g., propylene glycol, polyethylene glycol), non-ionic surfactant [e.g., polysorbate 80, HCO-50 (polyoxyethylene (50 mol) adduct of hydrogenated castor oil)] and the like. As examples of oily solutions, sesame oil, soybean oil and the like can be used, which may be used in combination with benzyl benzoate, benzyl alcohol and the like as solubilizers. The prepared injection solution is preferably filled in an appropriate ampoule. Suppositories used for rectal administration may be prepared by mixing the above-described antibody in an ordinary suppository base.

As the composition for oral administration, solid or liquid dosage forms, specifically tablets (including sugar-coated tablets and film-coated tablets), pills, granules, powders, capsules (including soft capsules), syrups, emulsions, suspensions and the like can be mentioned. Such a composition is produced by a publicly known method, and may contain a carrier, diluent or excipient in common use in the field of pharmaceutical making. As the carrier or excipient for tablets, lactose, starch, sucrose, and magnesium stearate, for example, are used.

The above-described pharmaceutical composition for parenteral or oral administration is conveniently prepared in a medication unit dosage form suitable for the dose of the antibody. As examples of such a medication unit dosage form, tablets, pills, capsules, injections (ampoules), and suppositories can be mentioned. It is preferable that the antibody be contained normally at 5 to 500 mg, particularly at 5 to 100 mg for injections or 10 to 250 mg for other dosage forms, per medication unit dosage form.

The dose of the above-described pharmaceutical containing the above-described antibody varies depending on the recipient of administration, target disease, symptoms, route of administration and the like; the pharmaceutical is administered by intravenous injection usually at about 0.01 to 20 mg/kg body weight, preferably about 0.1 to 10 mg/kg body weight, more preferably about 0.1 to 5 mg/kg body weight, based on a single dose of the antibody, several times at a frequency of once every 1 to 2 weeks, or once every 2 to 3 weeks for about 2 months. In case of other modes of parenteral administration and oral administration, similar doses may be administered. In case the symptom is particularly severe, the dose may be increased according to the symptom.

In another preferred embodiment, the B-iPS cell of the present invention can be utilized for generating an immunologically humanized mouse by being differentiated into a hematopoietic or immune system cell, and then transplanted to an immunodeficient mouse. Examples of the immunohematological system cell include, but are not limited to, hematopoietic stem cells, multipotent progenitor cells and the like. The cell need not to be a population of cells homogenous with respect to differentiation stage, and may be a heterogenous population of cells. Examples of methods of inducing the differentiation of a B-iPS cell into hematopoietic stem cells, and further into a B cell series, include, but are not limited to, a method described in JP-A-2006-141356 and the like.

Examples of preferred immunodeficient mice for use as the recipient include severe combined immunodeficiency mice (SCID mice) that lack the potential for producing T cells and B cells, particularly NOD/SCID/β2 microglobulin knock-out mice (NOD/SCID/B2M), NOD/SCID/common γ-chain knock-out mice, which do not have NK cell activity, and the like. Preferably, fetuses and neonates within 7 days after delivery, more preferably neonates within 2 days after delivery, are used as the recipient.

After a recipient mouse animal is systemically irradiated with radiation in advance, immunohematological system cells prepared in a specified amount are transplanted to the mouse. The number of cells to be transplanted can be determined as appropriate according to the mouse line, age and the like; for example, 1×10³ cells or more, preferably 1×10⁵ to 1×10⁷ cells, per animal can be transplanted.
For further details on generating an immunologically humanized mouse, Japanese Domestic Re-publication of PCT International Publication for Patent Application 2004-110139, for example, can be referenced to.

Immunologically humanized mice generated as described above are useful in that, for example, they make it possible to obtain information on the drug effect and/or adverse reactions to the human immune system of pharmaceutical candidate compounds that act on the immune system, in the preclinical phase. In model studies using conventional mice or monkeys, it is impossible to make a direct evaluation of effects on the human immune system. Among drugs that act on the immune system, not a few are totally ineffective on humans despite its efficacy in laboratory animals, or cause serious adverse reactions in humans; therefore, it is highly advantageous that preliminary findings concerning drug effects and adverse reactions in humans are obtained prior to clinical studies.

The present invention is hereinafter described in more detail by means of the following Examples, to which, however, the invention is not limited in any way.

### [Examples]

### Example 1: Establishment of iPS cells from mouse splenocyte-derived B cells

By a conventional method, B cells were prepared from splenocytes of a C57BL/6 mouse (purity 70%). The B cells were cultured in the presence of IL-2 (10 ng/ml) at a cell density of 10⁶ cells/ml, using an RPMI medium containing 10% FCS for 24 hours, after which the cells were infected with a retrovirus containing four mouse-derived factors (nucleic acids that encode Oct3/4, Sox2, Klf4, and c-Myc) (10⁶ pfu/ml) according to the method described in Cell, 126: 663-676 (2006) for 24 hours. After the viral infection, the cells were recovered, re-seeded onto mouse embryonic fibroblasts (MEF), and co-cultured in the presence of LIF using an ES cell culture medium. The emerging colonies were morphologically evaluated, and colonies assuming an ES-like morphology were picked up and further cultured in the presence of LIF on MEF, whereby 6 clones of B cell-derived iPS cells (B-iPS cells) were established (clone code names: 9a, 9b, 9c, 9d, 9e, 9f).

### Example 2: Characterization of B-iPS cells

To demonstrate that the 6 clones of B-iPS cells established in Example 1 were derived from B cells, whether rearrangement to B cell receptor (BCR) had occurred was determined by genomic PCR. Since each B cell had BCR already rearranged therein, PCR was performed using the primers shown below, with the genome of each B-iPS clone as the template, to confirm the presence or absence of the rearrangement.

### <Sense primers>

D_{HL}: MTTTTTGTSAAGGGATCTACTACTGTG (SEQ ID NO:1)
J_{H3}: CTCACAAGAGTCCGATAGACCCTGG (SEQ ID NO:2)
V_{β10}: TCCAAGGCGCTTCTCACCTCAGTC (SEQ ID NO:3)
V_{β5}: CCCAGCAGATTCTCAGTCCAACAG (SEQ ID NO:4)
V_{β8}: GCATGGGCTGAGGCTGATCCATTA (SEQ ID NO:5)
V_{H7183}: GAASAMCCTGTWCCTGCAAATGASC (SEQ ID NO:6)
V_{J558}: CARCACAGCCTWCATGCARCTCARC (SEQ ID NO:7)
V_{HQ52}: ACTGARCATCASCAAGGACAAYTCC (SEQ ID NO:8)

### <Antisense primers>

D_{β1}: TTATCTGGTGGTTTCTTCCAGC (SEQ ID NO:9)
D_{β2}: GCACCTGTGGGGAAGAAACT (SEQ ID NO:10)
J_{β1.5}: CAGAGTTCCATTTCAGAACCTAGC (SEQ ID NO:11)
J_{β2.6}: TGAGAGCTGTCTCCTACTATCGATT (SEQ ID NO:12)

As a result, it was confirmed that out of the 6 clones established, 3 clones (9a, 9b, 9f) had D_{H}-J_{H} gene recombination occurring therein, and 4 clones (9a, 9b, 9e, 9f) had VDJ recombination occurring therein (Fig. 1). Hence, it was confirmed that iPS cell could be established from B cells as well using the four factors (Oct3/4, Sox2, Klf4, c-Myc) only.

### Example 3: Establishment of iPS cells from purified mouse B cells

Splenocytes collected from a C57BL/6 mouse were stained with FITC-conjugated anti-CD19, and CD19-positive B cells were purified by MACS (Miltenyi Biotec Company) using anti-FITC beads.

The mature B cells obtained were cultured in the presence of IL-4 (10 ng/ml) and LPS (25 µg/ml) at a cell density of 10⁶ cells/ml, using an RPMI medium containing 10% FCS for 24 hours, after which the cells were infected with a retrovirus containing four mouse-derived factors (nucleic acids that encode Oct3/4, Sox2, Klf4, and c-Myc) (10⁶ pfu/ml) according to the method described in Cell, 126: 663-676 (2006) for 24 hours. After the viral infection, the cells were recovered, re-seeded onto mouse embryonic fibroblasts (MEF), and co-cultured in the presence of LIF using an ES cell culture medium. The emerging colonies were morphologically evaluated, and colonies assuming an ES-like morphology were picked up and further cultured in the presence of LIF on MEF, whereby iPS cells derived from the mature B cells was established. Shown in Fig. 2(a) is an outline of the procedure. An example of the mature B cell-derived iPS cell obtained is shown in Fig. 2(b).

Subsequently, the mature B cell-derived iPS cell obtained was injected into an embryo of a white murine; as shown in Fig. 2(c), a mouse wherein tissues derived from the iPS cell injected are co-present (chimeric mouse) could be generated. The B cell used to establish the above-described iPS cell had been isolated from a black mouse; in the mouse of Fig. 2(c), the black portion is thought to be derived from an iPS cell. This result shows that the established iPS cell possesses totipotency.

### Example 4: Establishment of iPS cells from antigen-specific B cells

As shown in Fig. 3(a), C57BL/6 mice were immunized by intraperitoneal administration of 100 µg of NP-CG/Alum per animal; 1 week later, splenocytes were collected. T lineage cells (anti-Thy1.2, anti-CD3, anti-NK1.1), myeloid lineage cells (anti-Gr1), antibody-producing cells (anti-CD138), and Ig κB cells (anti-Igκ) were removed from the splenocytes obtained, using MACS beads. Furthermore, using a cell sorter, B220-positive Igλ-positive NIP (immunizing antigen)-positive cells were isolated as antigen-specific B cells (Fig. 3(b)). iPS cells were established by the same procedure as Example 3, using the antigen-specific B cells obtained.
Subsequently, the DNA of the established iPS cells was sequenced. Among immunoglobulin genes of NP antigen-specific B cells, a combination of the H-chain V region V186.2 and the light-chain 1-chain is specifically abundant. Therefore, provided that an analysis of the immunoglobulin genes of the genome of the established iPS cell clone reveals rearrangement of the H-chain V region V186.2 and the light-chain 1-chain, the established clone can be regarded as being derived from the NP antigen-specific B cells.
The results of the sequencing showed that, in the iPS cell clone i56H1#12, for example, the H-chain had the V186.2 region rearranged therein, and the L-chain had the κ-chain rearranged therein but was of the type unable to produce protein due to an incorrect frame (unproductive type), whereas the λ-chain rearrangement occurred with a correct frame. Therefore, it can be concluded that the iPS cell clone i56H1#12 is of iPS cells derived from the antigen-specific B cells.

### Reference Example 1: Generation of antibody-producing cells from iPS cells

As the procedure is shown in Fig. 4(a), B cells were induced from iPS cells. Specifically, induction was performed as described below. Mouse mesenchymal cell-derived iPS cells were seeded at 5×10⁴ cells per plate of OP9 stromal cells. Six days later, the cells were detached and recovered with Trypsin-EDTA, and re-seeded onto fresh OP9 stromal cells. At that time, Flt-3L was added at a concentration of 5 ng/ml. After further cultivation for 4 days, the cells on the stromal cells were recovered by pipetting, and re-seeded onto fresh OP9 stromal cells. At that time, Flt-3L and IL-7 were added at concentrations of 5 ng/ml and 1 ng/ml, respectively. The cells were further cultured for 10 days. The FACS profile of the cells thus obtained is shown in Fig. 4(b). The FACS profile reveals the presence of a population of IgM-positive cells in the population of B220-positive cells, confirming the induction of B cells.
Subsequently, to induce antibody-producing cells from the B cells obtained, the B cells were stimulated with LPS (25 mg/ml) and IL-4 (10 ng/ml). Shown in Fig. 4(c) are FACS profiles obtained without stimulation (left in the lower panel) and with stimulation (right in the lower panel), respectively; it is seen that CD138-positive antibody-producing cells were obtained by stimulation with the antigens. Note that CD138 is a marker of antibody-producing cells (plasma cells). Shown in Fig. 4(d) is an example of antibody-producing cells (plasma cells) obtained from an iPS cell.
These results confirmed that induction of B cells (IgM-positive cells, plasma cells) from an iPS cell is possible. This leads to the expectation that by using the B-iPS cell of the present invention, which has been immunized with a specified antigen, it is possible to produce a monoclonal antibody against the antigen efficiently.

### Industrial Applicability

According to the B-iPS cell of the present invention, a human monoclonal antibody can be produced without using gene engineering technology, so that an antibody pharmaceutical can be provided at extremely low cost; the present invention is useful in that the market for antibody pharmaceuticals, which are currently biased to intractable diseases such as cancers, can be expanded to cover common diseases. Also, according to a humanized mouse generated by utilizing the B-iPS cell of the present invention, it is possible to evaluate the direct effect and adverse reactions to the human immune system of a drug that acts directly on the immune system, in the preclinical phase, so that the present invention is also useful in determining whether development of a pharmaceutical candidate compound is to be continued.

This application is based on a patent application No. 2008-227325 filed in Japan (filing date: September 4, 2008), the contents of which are incorporated in full herein.

## Claims

1. A cloned cell obtained by contacting a B cell with nuclear reprogramming factors that do not include C/EBPα and Pax5 expression inhibiting substances, wherein the cloned cell has immunoglobulin genes rearranged therein and possesses pluripotency and replication competence.

2. The cell according to claim 1, wherein the nuclear reprogramming factors are Oct3/4, Sox2, Klf4 and c-Myc or nucleic acids that encode the same, or Oct3/4, Sox2 and Klf4 or nucleic acids that encode the same.

3. The cell according to claim 1 or 2, wherein the B cell is of human derivation.

4. The cell according to any one of claims 1 to 3, wherein the B cell is immunized with a specified antigen.

5. A method of producing a monoclonal antibody against a specified antigen, comprising recovering an antibody from a culture of B cells obtained by differentiating the cell according to claim 4.

6. A method of generating an immunologically humanized mouse, comprising transplanting human immunohematological system cells obtained by differentiating the cell according to claim 3 to an immunodeficient mouse.

7. An immunologically humanized mouse obtained by the method according to claim 6.
